(19) Europäisches Patentamt
European Patent Office
Office européen des brevets

(11) EP 2 380 609 B1

(12) **EUROPEAN PATENT SPECIFICATION**

(45) Date of publication and mention
of the grant of the patent:
**07.06.2017 Bulletin 2017/23**

(51) Int Cl.:
***A61M 1/16*** *(2006.01)*

(21) Application number: **10004270.4**

(22) Date of filing: **22.04.2010**

(54) **Devices and controller for controlling of a filtration rate**

Geräte und Steuergerät zur Steuerung einer Filtrationsrate

Dispositifs et contrôleur pour contrôle de la vitesse de filtrage

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(43) Date of publication of application:
**26.10.2011 Bulletin 2011/43**

(73) Proprietor: **Fresenius Medical Care Deutschland
GmbH
61352 Bad Homburg (DE)**

(72) Inventors:
• **Chamney, Paul
Longfield Road, Tring
HP23 4DZ (GB)**
• **Moissl, Ulrich
61184 Karben (DE)**
• **Wabel, Peter
64287 Darmstadt (DE)**
• **Wieskotten, Sebastian
64560 Erfelden (DE)**

(74) Representative: **Bobbert & Partner
Patentanwälte PartmbB
Postfach 1252
85422 Erding (DE)**

(56) References cited:
**EP-A2- 1 927 371         WO-A1-98/23311
WO-A1-2004/022135   WO-A1-2007/140993
WO-A2-02/47609         US-A1- 2007 215 545**

**Description**

The present invention relates to an apparatus comprising a controller for controlling of a filtration rate during treatment of a bodily fluid of a patient by means of a bodily fluid treatment device according to claim 1. The invention also relates to a device.

Quite frequently, patients have to be treated regarding to their fluid balance by means of bodily fluid treatment devices, e.g., a dialysis apparatus. From WO 02/47609 A2 a feedback control of ultrafiltration is known to prevent Hypotension using osmotic pressure. From WO 2004/022135 A1 a control apparatus and a control method for a blood treatment equipment is known. From WO 2007/140993 A1 a device and method is known for controlling an extracorporeal blood treatment device. From WO 98/23311 A1 a method and system for preventing intradialytic symptomatology is known. From US 2007/0215545 A1 an extracorporeal renal replacement modelling system is known. From EP 1 927 371 A2 a method is known for increasing the safety of a blood processing device.

The present invention aims at providing an apparatus having a controller for controlling a filtration rate during dialysis of the patient. Also, the present invention suggests devices applicable in the field or context of filtration rate control.

By means of the present invention a controller for controlling a filtration rate during bodily fluid treatment by means of a device such as a device for blood treatment or dialysis is suggested. Also, a device for treating a patient's blood are provided.

The apparatus according to the invention is defined by the feature combination of claim 1.

The patient can be either a human being or an animal. The patient may be sane or ill. The patient may be in need of medical care or not. The patient may be a dialysis patient or not.

The controller comprises means needed and suited and/or configured to carry out the respective steps of the methods described herein.

In particular, the controller comprises a target relation defining means programmed for providing a pre-set target relation between one or more calculated or measured value(s) reflecting the mass or the concentration of a substance comprised by a tissue or a bodily fluid of the patient, and one or more calculated or measured value(s) reflecting an approximation of the distribution space of the patient as contained in the database; wherein the approximation of the distribution space of the patient is based on either measured or calculated values reflecting the overhydration or the relative overhydration of the patient; a calculation means configured for, during treatment of the bodily fluid, repeatedly calculating of value(s) reflecting the mass or the concentration of the substance and/or reflecting an approximation of the distribution space, and determining the relation there between at least once; and a signal output means configured for outputting of one or more signals to a controlling means for controlling the filtration rate of the fluid treatment device such that the determined relation is or approaches the target relation.

The apparatus according to the invention comprises means for obtaining one or more value(s) reflecting or concerning the distribution space or an approximation or changes thereof of the patient's body, and/or means for obtaining a value reflecting the mass or the concentration of the substance or changes thereof. The apparatus further comprises at least one controller according to the invention and a database.

The device according to the invention is defined by the feature combination of claim 7. It comprises at least one apparatus according to the invention.

Embodiments can include one or more of the following features.

In certain embodiments, the target relation is defined by a target range or one or more target line(s).

In some embodiments, the target range is a combination of one or more thresholds.

In certain embodiments, the target line or range is a trajectory in a diagram, preferably in a diagram in which a no-refill-curve might also be or is illustrated. Also, preferably, the target line or range is a trajectory in a diagram depicting a value representing the mass or the concentration of a substance (or changes thereof) as referred to herein over a distribution space (or changes thereof) - or vice versa.

In some embodiments, the target relation is defined before starting the respective dialysis treatment. In certain embodiments, the target relation is defined during the respective dialysis treatment.

In some embodiments, the controlling of a filtration rate during dialysis is an intentional step. In these embodiments, the result of the control as regards the filtration rate is not to be mixed up with results achieved by chance or at random or in an uncontrolled manner.

In certain embodiments, the controller or another device according to the invention comprises means for determining or measuring the blood volume BVo in the normohydrated condition (i.e., with a relative overhydration of about 0 liter) of the patient.

In some embodiments, the blood volume BVo can be determined by a body composition measurement using the following equation:

$$BV_0 = 0.1 \times LTM + 0.01 \times ATM \tag{1}$$

with LTM being the lean tissue mass and ATM being the adipose tissue mass. Both LTM and ATM can be measured with monitors available in the market.

In certain embodiments, the blood volume BVo can be determined by means of a anthropometric blood volume equation. In certain embodiments, the following formula by Nadler et al. is used:

For males:

$$BVo = 0.3669 \times (\text{height of the person at issue in meter})^3 + 0.03219 \times \text{weight of that person in kg} + 0.6041 \tag{1a}$$

For females:

$$BVo = 0.3561 \times \text{height}^3 \text{ (in meter)} + 0.03308 \times \text{weight in kg} + 0.1833 \tag{1b}$$

as published in Nadler, S.B., Hidalgo, J.U. and Block, T.: "Prediction of Blood Volume in Normal Human Adults." Surgery, 51, 224-232, 1962.

In some embodiments, the controller or another device according to the invention comprises means for determining or measuring a maximum-refill curve or steady-state curve of the patient. In certain embodiments, the maximum-refill curve or steady-state curve is one boundary or limit of the target range for the relation.

The course of the maximum-refill curve or steady-state curve of the patient is in certain embodiments calculated or identified as follows:

The concentration c_Hb (also referred to in the following equations as "Hb") of haemoglobin (Hb) depends on the mass m_Hb (or $m_{Hb}$) of Hb and the present blood volume BV of a given point of time as follows:

$$Hb = \frac{m_{Hb}}{BV} \tag{2}$$

BV in turn is the sum of the blood volume BVo at normohydrated conditions and the overhydration volume stored within the blood vessel system. The latter can be calculated by means of the slope of the Guyton curve being a curve depicting the blood volume BV over the extracellular fluid volume ECW of a patient explaining physiologic interdependencies between extracellular water (ECW) and the blood volume. The overhydration volume stored within the blood vessel system equals K_Guyton * OH, with K_Guyton being the slope of the Guyton curve:

$$Hb = \frac{m_{Hb}}{BV_0 + k_{Guyton} \times OH} = \frac{1}{\frac{BV_0}{m_{Hb}} + \frac{k_{Guyton}}{m_{Hb}} \times OH} \tag{3}$$

That way, the relation between concentration c_Hb of Hb and the overhydration OH can be expressed by

$$Hb = \frac{1}{b + a \times OH} \quad \text{with } b = \frac{BV_0}{m_{Hb}} \text{ and } a = \frac{k_{Guyton}}{m_{Hb}} \tag{4}$$

Hence, by assessing the patient's haemoglobin concentration c_Hb over overhydration OH or over relative overhydration relOH before starting a dialysis treatment, one will get data that can be used for drafting or approximating the steady-state-curve or maximum-refill curve of that patient before starting a dialysis treatment or at the beginning thereof. As is obvious, the course of that curve can be drafted the more precisely the more constant m_Hb of Hb is for that patient. Also, the steady-state-curve or maximum-refill curve is more precise in the linear sections of the Guyton curve.

As can be seen from equation (4), from the approximated parameter b and by means of BVo, the total mass m_Hb of Hb can be determined; also, from parameter a and the total mass m_Hb of Hb the slope K_Guyton of the curve can be determined.

It is noted that the steady-state curve can be determined, approximated or drawn once two data gained or measured independently from each other or at different time points are known as well as BVo. In all other cases, as few as three data gained form different measurements will do as well.

In certain embodiments, the course of the steady-state curve is approximated based on only two measurement data as follows:

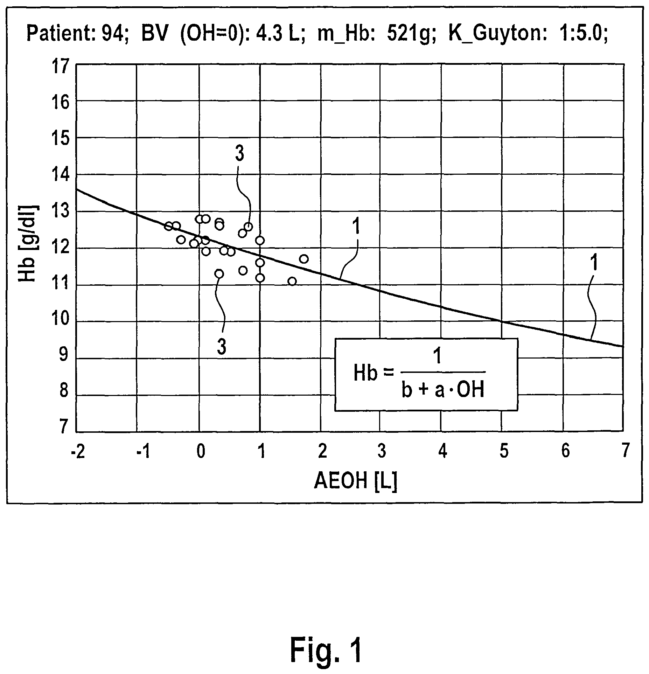

Before dialysis treatment, AEOH_pre and Hb_pre are measured (e.g. by means of monitors commercially available from the present applicant). In certain embodiments, during dialysis treatment, a sufficiently strong UF bolus - that is, a suddenly increased ultrafiltration rate - is applied. In sufficient time before the dialysis treatment is terminated, the ultrafiltration is terminated for achieving a stable value called Hb_post indicating a Hb concentration. Once the refill process has been awaited and taken place, a value for AEOH_post is calculated by means of the ultrafiltration volume UFV. The steady-state curve (as is shown, e.g., in Fig. 1) can be approximated based on AEOH_pre, AEOH_post, Hb_pre, and Hb_post.

In some embodiments, a number of minor boluses is applied yielding the same result as is achieved with one strong bolus.

In certain embodiments, no bolus is applied at all. For example, if the filtration rate is set such that during filtration a maximum refill is achieved, the course of the treatment takes place just on the steady state curve.

In some embodiments, the course of the steady-state curve is approximated based on only two arbitrarily chosen measurement data. One exemplary procedure in which the curve is approximated on just two measurement data can be described as follows:

Before dialysis treatment, AEOH_pre is measured. During dialysis treatment, a rather low ultrafiltration rate is set such that a refill process can be observed or achieved from the onset or the very beginning of the dialysis. From the present ultrafiltration volume UFV and AEOH_pre, the present value for AEOH can be calculated. The present value for Hb can be determined by means of the blood volume monitor.

In some embodiments, the at least one value reflecting the mass or the concentration of the substance was obtained from blood samples.

In certain embodiments, the substance is comprised by the group comprising at least any protein produced naturally in the body of the patient, in particular haemoglobin (short: Hb), albumin, insulin, glucose, c-reactive protein (short: CRP), and non endogeneous substances, in particular pharmaceutically effective substances.

In some embodiments, the mass or the concentration of the substance or changes thereof is an indicator of the anaemia state of the patient.

In certain embodiments, the anaemia state of the patient is defined by means of direct or indirect measurements or calculations of the mass or the concentration of a substance, e.g. haemoglobin (Hb), or changes over time thereof.

In some embodiments, the anaemia state of the patient is defined by means of direct or indirect measurements or calculations of the haematocrit (Hct) or changes over time thereof.

In some embodiments, the distribution space of the patient is an either measured or calculated value of the blood volume (BV). In certain embodiments, the distribution space is the blood volume at the beginning of another treatment session including filtration of the blood. In some embodiments, the distribution space is the intravascular blood volume. In certain embodiments, the distribution space and/or the blood volume encompasses the volume of the tube system of an extracorporeal blood system or parts thereof that is/are filled with blood. In certain embodiments, those parts may amount to 100, 150 or more millilitre (ml). For example, those parts comprise 130 ml with the applicant's dialysis device of the 5008 type (and 170 ml when used for Single Needle applications), and 170 ml with the applicant's dialysis device of the 2008 or 4008 type (and 210 ml when used for Single Needle applications). Also, in some embodiments, the blood capacity of the filter is also considered as part of the blood volume and/or the distribution space. The blood volume comprised by the filter may amount to 74 ml (FX60 by Fresenius, Germany) or about 100 ml (FX80 by Fresenius, Germany).

In certain embodiments, the distribution space is defined as the ECW (extracellular water), the extracellular volume or fluid, the ICW (intracellular water), the intracellular volume or fluid, the plasma volume, the TBW (total body water), the liquor, the volume of oedema, lymph, urine, or any other bodily fluid or volume, and also combinations thereof. Also, the distribution space within the meaning of the present invention can be any ratio of volumes as mentioned before, e.g. ECW/ICW, etc.

In some embodiments, a target range for the target relation is defined in a diagram reflecting the mass or the concentration of the substance and the distribution space or the approximation thereof.

In certain embodiments, the controller or another device comprises means for calculating a no-refill-curve.

In some embodiments, values that lay on the no-refill-curve are considered as a edge of a target range for the target relation.

In certain embodiments, the no-refill-curve relates to the state (which may change during dialysis and depending on the relative overhydration of the patient treated by means of dialysis) in which the blood volume is decreased by the same amount as is the (ultra)filtration volume (per time unit). As a result, the blood volume BV decreases quickly, and the Hb concentration c_Hb increases in turn. The no-refill-curve can delimit a no-refill-area (in a figure, for example) or no-refill-condition (in a patient, for example) from a refill-area or a refill-condition.

In some embodiments, the controller or another device according to the invention comprises means for calculating and/or measuring parameters reflecting the mass or the concentration of the substance and/or the distribution space of the patient or an approximation thereof.

In some embodiments, the controller or another device further includes means for assessing the size of at least one distribution space based on measured values and/or results of calculations reflecting a haemoglobin (Hb) state. The haemoglobin (Hb) state may be reflected by the Hb concentration, its total mass, its volume, change thereof over time, respectively, etc.

In certain embodiments, the controller or another device further includes means for assessing the size of at least one distribution space based on measured values and/or results of calculations reflecting the haematocrit (Hct) or changes thereof over time.

As is evident to the skilled person, the assessment of the size of at least one distribution space is not limited to be based on measured values and/or results of calculations reflecting a haemoglobin (Hb) state or the haematocrit (Hct) or changes thereof. The invention can of course also be carried out based on measured values and/or results of calculations reflecting a mass or concentration (and changes thereof) of any other suitable substance or marker.

In some embodiments, the controller or another device further includes means for using the size of at least one distribution space as obtained based on results from measurement of blood samples and/or from blood comprised in extracorporeal blood lines by means of an appropriate monitor. The measurements can be made by measuring the optical properties of the blood by optical sensors and/or by assessing acoustic properties like transit times and/or propagation velocities of ultrasonic pulses by ultrasonic sensors.

For determining the hydration state also any appropriate monitor can be used, such as monitors based on bioimpedance or dilution techniques.

In certain embodiments, the controller or another device further includes means for using the size of at least one distribution space as obtained based on results from urine samples.

In some embodiments, the controller or another device further includes means for using the size of at least one distribution space as obtained based on results from tissue samples.

In certain embodiments, the controller or another device further includes means for plotting results of the control for visual assessment.

In certain embodiments, the apparatus for controlling of a filtration rate during dialysis comprises means for obtaining a value reflecting the distribution space or an approximation or changes thereof of the patient's body, and/or means for obtaining a value reflecting the mass or the concentration of the substance or changes thereof. In these embodiments, the apparatus comprises at least one controller according to the invention.

In some embodiments, the apparatus according to the invention comprises means for measuring or calculating the distribution space or an approximation or changes thereof, in particular for measuring or calculating the hydration state or an overhydration, or wherein the means for obtaining a value consists of such means for measuring or calculating.

In certain embodiments, the apparatus comprises means for obtaining a value reflecting the mass, the volume or the concentration of the substance that comprises at least one of weight means, means for determining the blood volume of the patient, a keyboard, a touch screen, means for measuring or calculating the concentration, the volume and/or the mass of the substance, in particular haemoglobin (Hb) in blood, or changes thereof, or wherein the means for obtaining a value consists of such means for measuring or calculating.

In some embodiments, the apparatus according to the invention comprises means configured and intended for determining or assessing the relation between values.

In certain embodiments, the apparatus is or comprises a monitor for obtaining information concerning the control.

In some embodiments, the means for measuring or calculating the distribution space or an approximation or changes thereof is a monitor as described in WO 2006/002685 A1. Of course, the present invention must not be understood to be limited to means or monitors obtaining data by bioimpedance measurements as is described in WO 2006/002685 A1. Other bioimpedance methods known in the art and also any other methods known in the art such as dilution measurements and also any other method known to the skilled person are also contemplated and encompassed by the present invention as well.

In certain embodiments, the apparatus comprises a means or monitor for measuring Hb concentrations (e.g., in [g/dl]) and/or for determining the blood volume by means of any monitor as described in "Replacement of Renal Function by Dialysis" by Drukker, Parson and Maher, Kluwer Academic Publisher, 5th edition, 2004, Dordrecht, The Netherlands, on pages 397 to 401 ("Hemodialysis machines and monitors"). In some embodiments, the means or monitor is configured to measure the blood volume and/or the concentration of the substance - in particular Hb - by means of measuring an electrical conductivity.

In certain embodiments, the means or monitor is configured to measure the blood volume and/or the concentration of the substance - in particular Hb - by means of measuring an optical density.

In some embodiments, the means or monitor is configured to measure the blood volume and/or the concentration of the substance - in particular Hb - by means of measuring a viscosity.

In certain embodiments, the means or monitor is configured to measure the blood volume and/or the concentration of the substance - in particular Hb - by means of measuring a density.

In some embodiments, the means or monitor comprises one or more corresponding probes and/or one or more sensors for carrying out the measurements such as electrical conductivity sensors, optical sensors, viscosity sensors, density sensors, and the like.

In certain embodiments, the apparatus furthermore comprises an output device for outputting results provided by the controller.

In some embodiments, the output device is a monitor having a display, a plotter, a printer or any other means for providing an output.

In certain embodiments, the output device is connected to an actuator for controlling administration of a substance to the patient.

In other embodiments, the device may be used for treating a patient (or the patient's blood) by haemofiltration, ultrafiltration, haemodialysis, etc.

The embodiments may provide one or more of the following advantages.

In some embodiments, the invention provides information on how the filtration rate should be controlled or fixed. The information can be advantageously used to prevent certain decreases of the patient's blood pressure caused by the dialysis. Also, the occurrence of nausea, convulsions, emesis, vertigo, impaired vision and other symptoms frequently caused by the filtration procedure can advantageously be reduced or avoided.

In certain embodiments, the invention provides information on how high the filtration rate may be set to both prevent unwanted blood pressure drops and to accomplish the dialysis treatment as quick as possible (i.e., so as not to waste the patient's time or not to bind him to the treatment site longer than required). The latter may happen the closer the relation is to relations forming a maximum-refill curve or steady-state curve as described above.

Other aspects, features, and advantages will be apparent from the description, figures, and claims.

In the following, the invention is further explained by means of the figures of the drawing wherein same or identical elements are identified by means identical reference numerals. However, the invention must not be understood to be limited to the examples explained by means of the figures.

Fig. 1    shows a steady-state curve depicting the relation between the Hb-concentration and the overhydration of a dialysis patient in a diagram;

Fig. 2    shows an intradialytic course of the relation between Hb-concentration and the overhydration during dialysis in the diagram of Fig. 1;

Fig. 3    additionally shows a no-refill curve in the diagram of Fig. 2;

Fig. 4    shows a first apparatus comprising a controller for carrying out the invention;

Fig. 5    shows a second apparatus comprising a controller for carrying out invention;

Fig. 6    shows a schematic cut through body tissue for defining certain terms relating to distribution spaces as used within the present specification;

Fig. 7    shows an example of how certain terms relating to distribution spaces may be understood in terms of the present invention and how certain distribution spaces may be interconnected by means of a general concept;

**Fig. 8**    shows the intradialytic course of a patient's haemoglobin/overhydration relation; and

**Fig. 9**    shows a threshold and a trajectory for a dialysis by means of ultrafiltration, illustrated in the diagram of Fig. 2.

**Fig. 1** shows a maximum-refill curve or steady-state curve 1 depicting the relation between the Hb-concentration (y-axis, Hb, in ["g/dl]") of an anonymized dialysis patient "94" and his overhydration (x-axis, AEOH, in [litres]) at the beginning of his fluid removal treatments. Curve 1 can be gained from curve-fitting of a number of measurements 3. Measurements 3 can be gained, e.g., from measurements by means of monitors as disclosed in WO 2006/002685 A1.

By means of BVo, which can be measured, and by means of the parameter b, which can be approximated from Fig. 1 (see also the equations reproduced above), the total mass of Hb, ("m_Hb") can be determined.

Patient "94" has a blood volume BV of 4.3 L when measured in the normohydration state with OH = 0. Further, it has been calculated that patient "94" has a total Hb mass m_Hb of 521 g.

By means of m_Hb and the parameter a, which can also be approximated or derived from Fig. 1, the slope K_Guyton of the patient's current position on the Guyton curve - with the Guyton curve being a curve depicting the blood volume BV over the extracellular fluid volume ECW of a patient explaining physiologic interdependencies between extracellular water (ECW) and the blood volume - can be determined. In Fig. 1, K_Guyton is around 1:5.0.

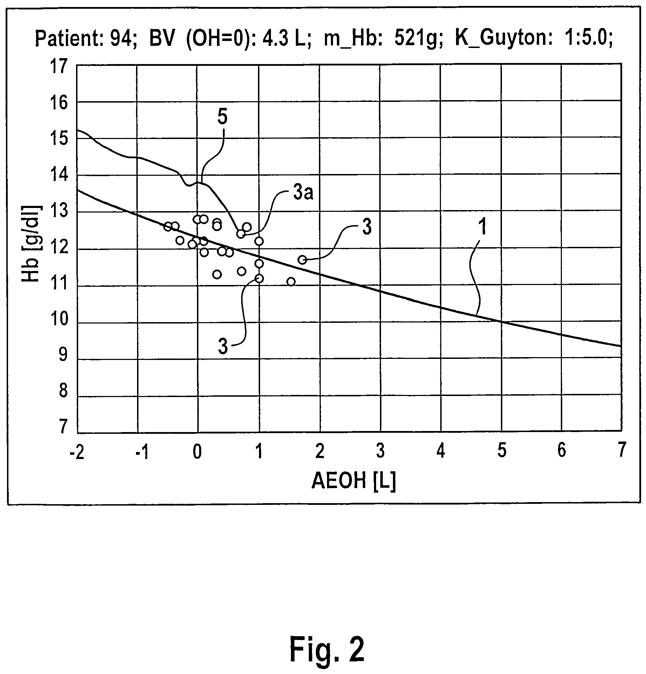

Fig. 2 shows an intradialytic course or development of the relation between the Hb-concentration c_Hb and the overhydration AEOH of patient "94" during dialysis. In most aspects, Fig. 2 is identical to Fig. 1.

Starting from one particular measurement 3a out of the measurements 3 shown both in Fig. 1 and Fig. 2, an intradialytic curve 5 can be established during dialysis of the patient.

Curve 5 reveals the relation between Hb concentration c_Hb and the overhydration AEOH during the dialysis treatment. Since curve 5 reflects a number of measured and approximated values for the relation, curve 5 also reveals changes of the relation over time.

In case of applying very low ultrafiltration rates during dialysis, curve 5 would be identical to steady-state revealing curve 1. However, this is not shown in Fig. 2. There, curve 5 clearly deviates from curve 1.

In case curve 5 is identical to curve 1, then the refill-processes which can effect a shift from water from the interstices to the vessels of the patient have enough time to happen. For that reason curve 1 can also be named a "maximum refill curve". Obviously, in Fig. 2 the ultrafiltration rate is set too high for allowing a total refill during the course of dialysis.

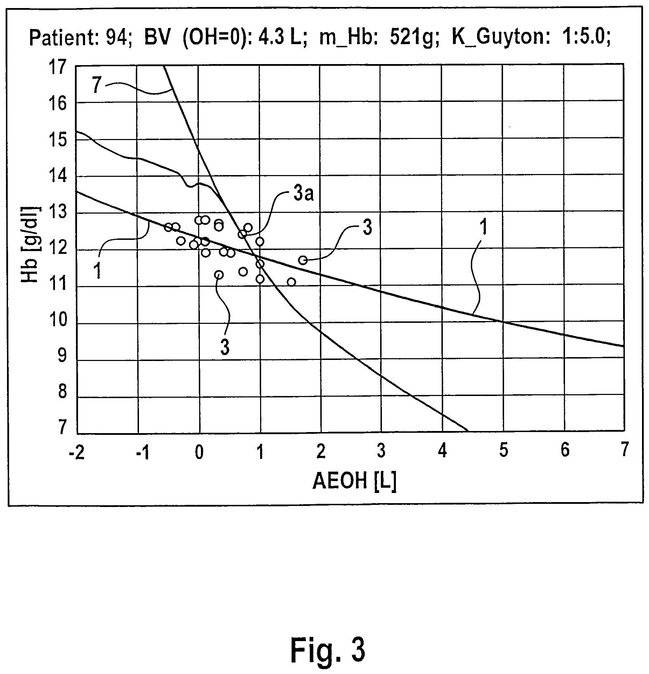

**Fig. 3** shows the diagram of Fig. 2 into which a "no-refill" curve 7, as already explained above, has been added.

The no-refill curve 7 can be calculated by means of

$$Hb = \frac{m_{Hb}}{BV_{Start} - UFV} \ , AEOH = AEOH_{pre} - UFV \tag{5}$$

with UFV being the ultrafiltration volume, AEOHpre being the overhydration or relative overhydration measured before starting the dialysis, and BVstart being the blood volume at starting the dialysis.

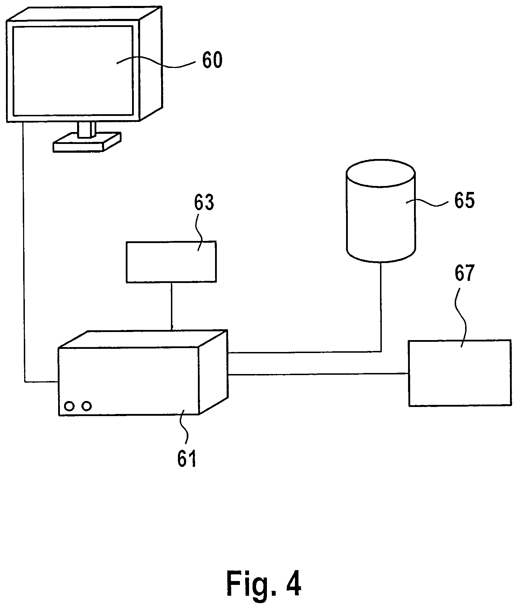

**Fig. 4** shows an apparatus 61 comprising a controller 63 according to the invention. The apparatus 61 is connected to an external database 65 comprising the results of measurements and the data needed. The database 65 can also be an internal means of the apparatus 61. The apparatus 61 may optionally have means 67 for inputting data into the controller 63 or into the apparatus 61 itself. Such data may be information about the size of one or more distribution spaces, the mass, the volume, the concentration of a substance as is set forth above, etc., or approximations thereof. Also, the criterion may be input by means of the input means 67. The criterion may, however, alternatively be stored in database 65 or any other storage means. The criterion may be calculated or determined by the controller 63 or any other item comprised by the apparatus 61 or interconnected to it. The results of the evaluation, calculation, comparison, assessment etc. performed by the controller 63 and/or the apparatus 61 can be displayed on a monitor 60 or plotted by means of a - not displayed but optionally also encompassed - plotter or stored by means of the database 65 or any other storage means.

In particular, the controller 63 can be configured for determining a value reflecting the distribution of the fluid, and for assessing whether the relation fulfils at least one criterion.

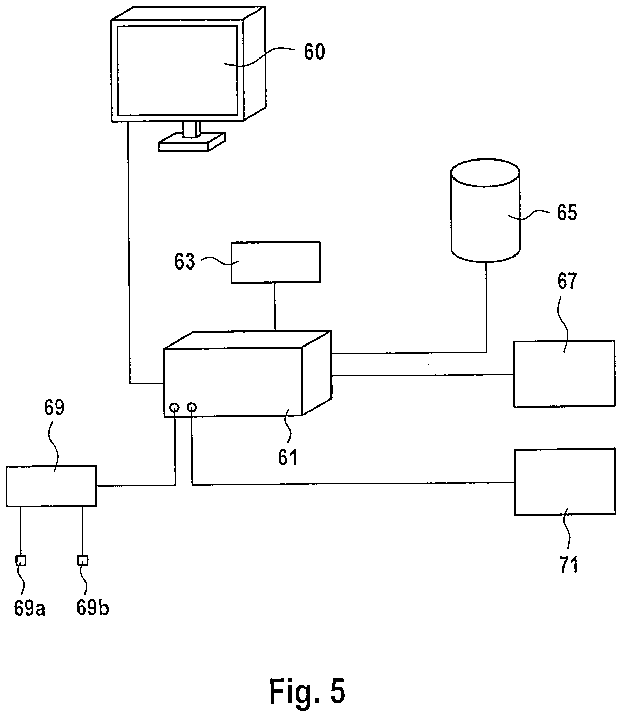

As can be seen from **Fig. 5,** for corresponding measurements, the apparatus 61 can be connected (by means of wires or wireless) with a bioimpedance measurement means 69 as one example of a means for measuring or calculating the distribution or the size(s) of one or more distribution spaces or approximations or changes thereof. Generally, the means for measuring or calculating can be provided in addition to the external database 65 comprising the results of measurements and the data needed for the present invention, or in place of the external database 65 (that is, as an substitute).

The bioimpedance measurement means 69 can be capable of automatically compensating for influences on the impedance data like contact resistances.

An example for such a bioimpedance measurement means 69 is a device from Xitron Technologies, distributed under the trademark Hydra™ that is further described in WO 92/19153. The bioimpedance measurement means 69 may comprise various electrodes. In Fig. 5, only two electrodes 69a and 69b are shown which are attached to the bioimpedance measurement means 69. Additional electrodes are, of course, also contemplated.

Each electrode implied can comprise two or more ("sub"-)electrodes in turn. Electrodes can comprise a current injection ("sub-")electrode and a voltage measurement ("sub-")electrode. That is, the electrodes 69a and 69b shown in Fig. 5 can comprise two injection electrodes and two voltage measurement electrodes (i.e., four electrodes in total).

Generally spoken, the apparatus according to the invention can be provided with means such as weighing means, a keyboard, a touch screen etc. for inputting the required data, sensors, interconnections or communication links with a lab, any other input means, etc. Similarly, the apparatus 61 may have further means 71 for measuring or calculating means for obtaining a value reflecting the distribution of another distribution space and/or for obtaining values reflecting the mass, the volume or the concentration of the substance that can be provided in addition to the external database 65 or in place of the external database 65 (that is, as an substitute).

The means 71 can be provided as a weighing means, a keyboard, a touch screen etc. for inputting the required data, sensors, interconnections or communication links with a lab, a Hb (or any other substance suitable for measuring, calculating or approximating the size of a distribution space) concentration probe, any other input means, etc.

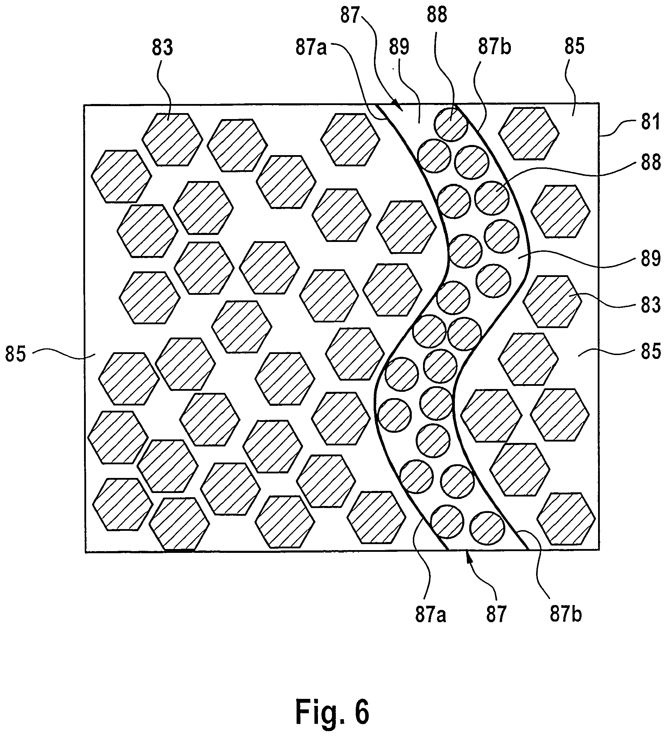

**Fig. 6** shows a schematic cut through body tissue for defining certain terms relating to distribution spaces as used within the present specification.

In particular, Fig. 6 shows a defined volume 81 comprising tissue cells 83 comprising intracellular water, an interstitium 85 comprising extracellular water, and a blood vessel 87 with cut vessel walls 87a and 87b.

The blood vessel comprises blood cells 88 (comprising intracellular water). The blood cells are embedded into blood plasma 89 (comprising extracellular water).

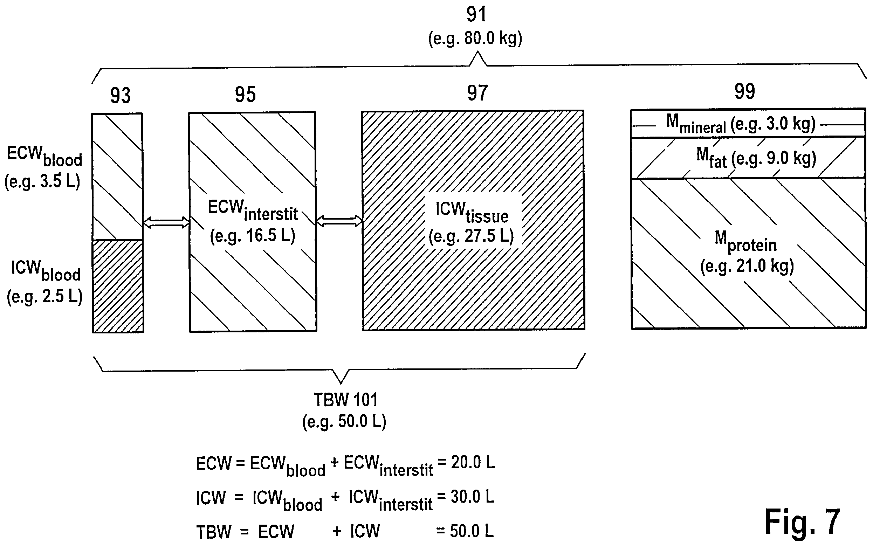

**Fig. 7** shows an example of how certain terms relating to distribution spaces may be understood in terms of the present invention. It also shows how certain distribution spaces may be interconnected by means of a general concept.

As can be seen from Fig. 7, the whole body weight 91 - which can amount to, for example, 80.0 kg - can be understood as the sum of the blood volume (BV) 93, the water in the interstitium (extracellular) (ECWinterstit) 95, the water in the cells of the tissue (intracellular) (ICWtissue) 97, and the solid components of the body 99.

The blood volume may be understood as the sum of the extracellular water that is present within the vessels (ECWblood), in particular in the blood plasma, and the intracellular water (ICWblood) that is present in the vessel or the (red) blood cells. In an example, the ECWblood may be 3.5 L, the ICW may be 2.5 L, the blood volume 93 may be 6.0 L.

The water in the interstitium (extracellular) 95 (ECWinterstit) can encompass 16.5 L. The water in the cells of the tissue (intracellular) 97 (ICWtissue) can encompass 27.5 L. The solid components 99 of the body comprise the mineral mass Mmineral which can have 3.0 kg, the fat mass Mfat which can be 9.0 kg, and the protein mass Mprotein which can amount to 21.0 kg. The solid components 99 can thus amount to 33.0 kg.

As can be seen from Fig. 7, the sum of the blood volume 93, the water in the interstitium (extracelluar) 95, and the water in the cells of the tissue (intracellular) 97 can be understood as the total body water 101 (TBW). The total body water 101 can encompass 50.0 L.

As can further be seen from Fig. 7, the sum of the total body water 101 and the solid components 99 can be equal to the whole body weight 91.

As is readily understood by the skilled one, the above given figures and weights are to be understood as examples which may be found in one particular patient, whereas other patients may reveal different weights and mass contributions. However, Figs. 6 and 7 are well suited for giving one example of how certain terms regarding to distribution spaces may be understood, and also how certain distribution spaces of a patient's body relate to each other.

Again, it is noted that all or at least some of the figures relate to Hb/anaemia state and volume or weight/hydration state by means of examples showing how one particular embodiment according to the invention may be carried out. They are not to be understood as limiting.

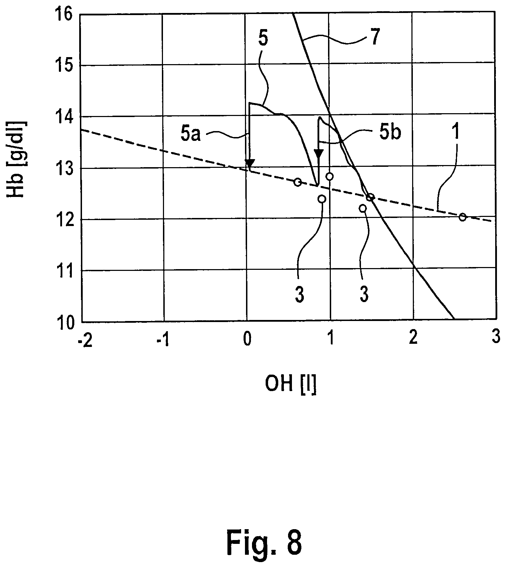

**Fig. 8** shows the intradialytic (i.e., during dialysis) development of a relation between haemoglobin concentration and overhydration of a particular patient, the development being identified as curve 5.

As can readily be understood, the course of the steady state revealing curve 1 can be determined by interrupting the treatment of the patient during one treatment session for a time span and continuing same, or by comparing data from several, at least two, treatment sessions. Following the interruption during the one treatment session or following the termination of the first treatment session out of several ones, the Hb concentration will decrease due to the refill-effect (as described above). In Fig. 8, such decrease can be observed twice and is depicted by means of reference numerals 5a and 5b. The time span may last until the Hb concentration does not change any more or at least that long.

As is evident, by means of two or more interruptions as described before, the steady state revealing curve 1 can be determined. Also, by means of one or more such interruptions, the validity of an earlier determined curve 1 can be assessed. For example, the course of the earlier determined curve 1 can be assessed by checking whether the decline of curve 5 (or portions 5a, 5b thereof, in particular) ends at or with an intersection of portion 5a or 5b (or similar portions which correspond to a decrease) with the steady state revealing curve 1.

Again, the idea explained with reference to Fig. 8 or any other figure attached hereto is not limited to Hb and OH. Any other parameter that might replace Hb and OH in the light of the present disclosure may also be used to find or confirm a steady state revealing curve.

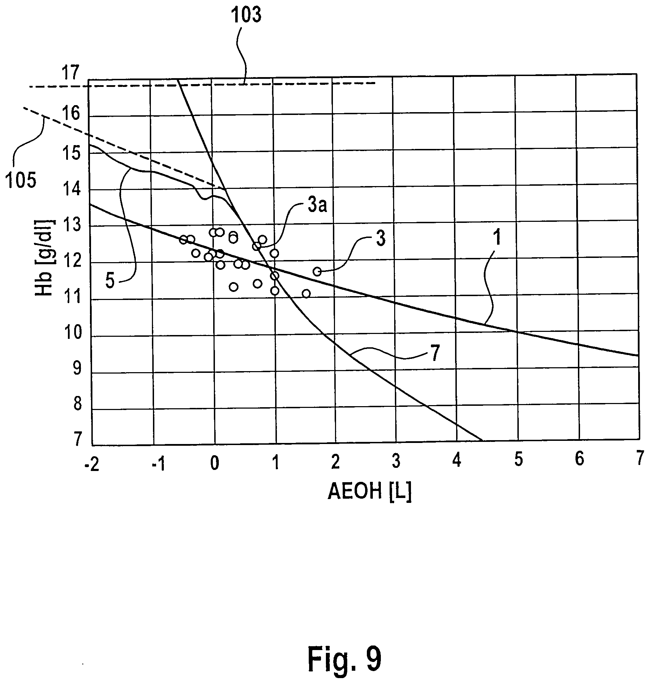

**Fig. 9** shows a threshold 103 and a trajectory 105, both being illustrated as broken or dashed lines, illustrated in the diagram known from Fig. 2 reflecting the course of an ultrafiltration treatment.

Both the threshold 103 and the trajectory 105 can be used - either together or taken alone - for controlling the treatment as is explained in the following example, the example being related to a body fluid treatment ultrafiltration.

As can be seen from Fig. 9, intradialytic ultrafiltration control can be improved by observing a maximum Hb concentration of the patient's blood during dialysis, the maximum Hb concentration being indicated by the threshold 103. In some embodiments, the dialysis process may be stopped once the intradialytic curve 5 indicating the intradialytic blood volume has crossed the threshold 103, is about to cross it or has crossed it for longer than a predetermined span of time, or the like.

If, for example, the minimum or minimum absolute blood volume BV should not drop below, say, 4 liter (L), the threshold 103 may set to 16.7 g/dl (or g(dL)) under the assumption that the mass of Hb is known, following from the facts that in the present example the mass of Hb amounts to 500 g, and that BVmin shall be 3 L or 30 dl (dL). Then, the maximum Hb concentration 16.7 g/dl is given by 500 g divided by 30 dl = 16.7 g/dl.

Also, a threshold can be chosen parallel to or in a certain, predetermined distance to the steady state revealing curve 1. The distance may be uniform or equidistant, or the distance may differ. The distance may vary depending on further data, measurements or values, including ones that also may vary. For example, the distance may be set to be a fixed value. The fixed value may be, e.g., 2 g/dl. In this example, the threshold runs 2 g/dl above curve 1.

Also, in certain embodiments, the dialysis process may be controlled such that the trajectory 105 is followed exactly, or as close as possible, or within a certain range or approximation or the like.

For example, in some embodiments, the dialysis process may be controlled such that the intradialytic curve 5 preferably follows the no-refill curve 7 during treatment for a certain time or a predetermined period of time, for example 30 min after the treatment has been started. If the intradialytic curve 5 does not leave the no-refill curve 7 (or stays at least within certain limits) or remains within a predetermined range given for the no-refill curve 7 within those 30 min, then the set ultrafiltration rate might be considered as appropriate. However, if the intradialytic curve 5 should behave differently, this may be taken as an indication that the ultrafiltration rate be better decreased.

In another attempt to control the ultrafiltration treatment, the ultrafiltration rate may be set such that a certain first trajectory (or a certain part thereof), which may, e.g., be in parallel to the no-refill curve 7, be followed before another trajectory or another part of the first trajectory, which may, e.g., run in parallel to the steady state revealing curve 1 or at a constant Hb concentration level, is followed. For example, the ultrafiltration rate may be set such that first the no-refill curve 7 is followed and later a trajectory such as trajectory 105 is followed.

In again another attempt to control the ultrafiltration treatment it may be ensured that the intradialytic curve 5 never exceeds an absolute Hb concentration limit which may be represented by a threshold such as the threshold 103, irrespective of the patient's blood volume, or does, if the limit is exceeded, meet certain conditions.

Besides, once the Hb concentration has risen from initial value by a set, fixed or predetermined difference of, e.g., 2 g/dl (for example from 11 g/dl to 13 g/dl), in certain embodiments ultrafiltration rate is controlled such that the Hb concentration is maintained at, for example, 13 g/dl. That implies constant blood volume BV with the ultrafiltration rate and the refill effect being balanced.

Finally, a critical blood volume, which may lead to the termination or interruption of the treatment if under-run, may be determined by making repeated blood pressure measurements during the treatment (e.g., dialysis). If the blood pressure should fall below a certain value, the blood volume existing at that point of time can be contemplated or set as the minimal or critical blood volume.

Again it is to be noted that the examples given above, in particular if based on the discussed figures, are not limited to the monitoring of Hb or OH during dialysis. Rather, above teaching and in particular the teaching of the figures is contemplated also for parameters other than Hb and/or OH, the parameter being monitored during any treatment, not only dialysis. In any case, the examples of the figures are chosen only for illustration purposes.

**Claims**

1.  An apparatus (61) for controlling a filtration rate during treatment of a bodily fluid of a patient, the apparatus (61) comprising:

    - means (69) configured for obtaining one or more value(s) reflecting the approximation or changes of a distribution space of the patient's body, and/or means for obtaining a value reflecting the mass, the volume or the concentration of a substance or changes thereof; and
- a controller (63);
- a database (65);

wherein the controller (63) is programmed to control the filtration rate during treatment of the bodily fluid by means of a bodily fluid treatment device, the controller (63) comprising

- a target relation defining means programmed for defining and providing a pre-set target relation between one or more calculated or measured value(s) reflecting the mass or the concentration of substance comprised by a tissue or a bodily fluid of the patient, and one or more calculated or measured value(s) reflecting an approximation of the distribution space of the patient as contained in the database (65); wherein the approximation of the distribution space of the patient is based on either measured or calculated values reflecting the overhydration (OH) or the relative overhydration (relOH) of the patient;
- a calculation means programmed for, during treatment of the bodily fluid, repeatedly calculating of value(s) reflecting the mass or the concentration of the substance and/or reflecting an approximation of the distribution space, and determining the relation there between at least once; and
- a signal output means programmed for outputting of one or more signals to a controlling means for controlling the filtration rate of the fluid treatment device such that the determined relation is or approaches the target relation.

2. The apparatus (61) according to claim 1, further comprising at least one means of

- a target defining means configured for defining a target range for the target relation in a diagram reflecting the mass or the concentration of the substance and the approximation of the distribution space; and/or
- a calculating means configured for calculating a no-refill-curve.

3. The apparatus (61) according to anyone of the preceding claims, wherein the substance is selected from the group consisting of any protein produced naturally in the body of the patient, haemoglobin, albumin, insulin, glucose, c-reactive protein (CRP), and non endogeneous substances, and pharmaceutically effective substances.

4. The apparatus (61) according to anyone of the preceding claims, wherein the mass or the concentration of the substance of changes thereof is an indicator of the anaemia state of the patient.

5. The apparatus (61) according to claim 4, wherein the substance is haemoglobin (m_Hb, Hb), its total mass or its change over time, or is the haematocrit (Hct) or changes over time thereof.

6. The apparatus (61) according to any of the preceding claims, wherein the means configured for obtaining a value reflecting the mass, the volume or the concentration of the substance comprises at least one of weight means, means for determining the blood volume of the patient, a keyboard, a touch screen, means for measuring or calculating the concentration, the volume and/or the mass of the substance, in particular haemoglobin (Hb) in blood, or changes thereof, or wherein the means for obtaining a value consists of such means for measuring or calculating.

7. A device for treating a patient's blood, comprising at least one apparatus according to anyone of the preceding claims.

8. The device according to claim 7, adapted for treating a patient by dialysis, haemofiltration, ultrafiltration, and/or haemodialysis.

**Patentansprüche**

1. Vorrichtung (61) zum Kontrollieren einer Filtrationsrate während der Behandlung eines Körperfluids eines Patienten, wobei die Vorrichtung (61) umfasst:

- Mittel (69) konfiguriert zum Erfassen eines oder mehrerer Werte, die die Näherung oder Veränderungen eines Verteilungsraums des Körpers des Patienten widerspiegeln, und/oder Mittel zum Erfassen eines Werts, der die Masse, das Volumen oder die Konzentration einer Substanz oder deren Veränderung widerspiegeln; und
- eine Steuerung (63);
- eine Datenbank (65);

wobei die Steuerung (63) programmiert ist, um die Filtrationsrate während der Behandlung des Körperfluids mittels eines Körperfluidbehandlungsgeräts zu steuern, wobei die Steuerung (63) umfasst:

- ein ein Zielverhältnis definierendes Mittel, das programmiert ist zum Definieren und Bereitstellen eines vorbestimmten Zielverhältnisses zwischen einem oder mehreren berechneten oder gemessenen Werten, die die Masse oder die Konzentration der Substanz widerspiegeln, die in einem Gewebe oder einem Körperfluid des Patienten enthalten sind, und einem oder mehreren berechneten oder gemessenen Werten, die eine Näherung des Verteilungsraums des Patienten wie in der Datenbank (65) enthalten widerspiegeln; wobei die Näherung des Verteilungsraums des Patienten auf entweder gemessenen oder berechneten Werten basiert, die die Überwässerung (OH) oder die relative Überwässerung (relOH) des Patienten widerspiegeln;
- ein Berechnungsmittel, das programmiert ist dazu, während der Behandlung des Körperfluids wiederholt die Werte, die die Masse oder die Konzentration der Substanz widerspiegeln und/oder eine Näherung des Verteilungsraums widerspiegeln, zu berechnen, und das Verhältnis zwischen diesen wenigstens einmal zu bestimmen; und
- ein Signalausgabemittel, das programmiert ist, ein oder mehrere Signal auszugeben an ein Steuermittel zum Steuern der Filtrationsrate der Fluidbehandlungsvorrichtung, so dass das bestimmte Verhältnis das Zielverhältnis ist oder sich diesem annähert.

**2.** Die Vorrichtung (61) nach Anspruch 1, zudem mindestens ein Mittel umfassend aus

- einem zieldefinierenden Mittel, das konfiguriert ist, um einen Zielwertebereich für das Zielverhältnis in einem Diagramm, das die Masse oder die Konzentration der Substanz und die Näherung des Verteilungsraums widerspiegelt; und/oder
- ein Berechnungsmittel, das konfiguriert ist, eine Nicht-Nachfüllen-Kurve zu berechnen.

**3.** Die Vorrichtung (61) nach einem der vorhergehenden Ansprüche, wobei die Substanz ausgewählt ist aus der Gruppe bestehend aus jedem Protein, das natürlich im Körper des Patienten hergestellt wird, Hämoglobin, Albumin, Insulin, Glukose, C-reaktives Protein (CRP) und aus nicht-endogene Substanzen und aus pharmazeutisch wirksamen Substanzen.

**4.** Die Vorrichtung (61) nach einem der vorhergehenden Ansprüche, wobei die Masse oder die Konzentration der Substanz oder Veränderungen davon ein Indikator für den Anämiestatus des Patienten sind.

**5.** Die Vorrichtung (61) nach Anspruch 4, wobei die Substanz Hämoglobin (m_Hb, Hb), seine Gesamtmasse oder seine Veränderung über die Zeit ist oder der Hämatokrit (Hct) oder seine Veränderung über die Zeit ist.

**6.** Die Vorrichtung (61) nach einem der vorhergehenden Ansprüche, wobei die Mittel, die konfiguriert sind zum Erfassen eines Wertes, der die Masse, das Volumen oder die Konzentration der Substanz widerspiegelt, mindestens eine(n) umfasst aus Gewichtsmittel, Mittel zum Bestimmen des Blutvolumens des Patienten, Tastatur, Touchscreen, Mittel zum Messen oder Berechnen der Konzentration, des Volumens und/oder der Masse der Substanz, insbesondere Hämoglobin (Hb) im Blut, oder Veränderungen davon, oder wobei die Mittel zum Erfassen eines Wertes aus solchen Mitteln zum Messen oder Berechnen besteht.

**7.** Gerät zum Behandeln des Bluts eines Patienten, umfassend mindestens eine Vorrichtung nach einem der vorhergehenden Ansprüche.

**8.** Das Gerät nach Anspruch 7, dazu eingerichtet, einen Patienten mittels Dialyse, Hämofiltration, Ultrafiltration und/oder Hämodialyse zu behandeln.

**Revendications**

**1.** Un appareil (61) pour contrôler un taux de filtration pendant le traitement d'un fluide corporel d'un patient, l'appareil (61) comprenant:

- des moyens (69) configurés pour obtenir une ou plusieurs valeur(s) reflétant l'approximation ou les changements d'un espace de distribution du corps du patient, et/ou des moyens pour obtenir une valeur reflétant la masse, le volume ou la concentration d'une substance ou des changements de ceux-ci; et

- un contrôleur (63);
- une base de données (65);

où le contrôleur (63) est programmé de façon à contrôler le taux de filtration pendant le traitement du fluide corporel au moyen d'un dispositif de traitement du fluide corporel, le contrôleur (63) comprenant:

- un moyen de définition d'une relation cible programmé afin de définir et de fournir une relation cible préétablie entre une ou plusieurs valeur(s) calculée(s) ou mesurée(s) reflétant la masse ou la concentration d'une substance comprise dans un tissu ou un fluide corporel du patient, et une ou plusieurs valeur(s) calculée(s) ou mesurée(s) reflétant une approximation de l'espace de distribution du patient comme contenu dans la base de données (65) ; où l'approximation de l'espace de distribution du patient est basée soit sur des valeurs mesurées ou calculées reflétant la surhydratation (OH) ou la surhydratation relative (relOH) du patient;
- un moyen de calcul programmé pour, lors du traitement du fluide corporel, calculer des valeurs reflétant la masse ou la concentration de la substance et/ou reflétant une approximation de l'espace de distribution de façon répétitive, et pour déterminer au moins une fois la relation entre celles-ci; et
- un moyen de sortie de signal programmé pour fournir un ou plusieurs signaux à un moyen de contrôle de façon à ce que la relation déterminée soit ou s'approche de la relation cible.

2. L'appareil (61) selon la première revendication, comprenant de plus au moins:

- un moyen de définition de cible configuré pour définir une fourchette cible pour la relation cible dans un diagramme reflétant la masse ou la concentration de la substance et l'approximation de l'espace de distribution; et/ou
- un moyen de calcul configuré pour calculer une courbe de non ré-remplissage.

3. L'appareil (61) selon l'une quelconque des revendications précédentes, où la substance est sélectionnée dans le groupe constitué de n'importe quelle protéine produite naturellement dans le corps du patient, de l'hémoglobine, de l'albumine, de l'insuline, du glucose, de protéines C réactives (CRP), ainsi que de substances non endogènes et de substances efficaces au niveau pharmaceutique.

4. L'appareil (61) selon l'une quelconque des revendications précédentes, où la masse ou la concentration de la substance, ou des variations de celles-ci, est un indicateur de l'état d'anémie du patient.

5. L'appareil (61) selon la revendication 4, où la substance est l'hémoglobine (m_Hb, Hb), sa masse totale ou sa variation en fonction du temps, ou l'hématocrite (Hct) ou des variations de celle-ci en fonction du temps.

6. L'appareil (61) selon l'une quelconque des revendications précédentes, où le moyen configuré pour obtenir une valeur reflétant la masse, le volume ou la concentration de la substance comprend au moins un moyen de pesée, un moyen prévu pour déterminer le volume sanguin du patient, un clavier, un écran tactile, un moyen prévu pour mesurer ou calculer la concentration, le volume et/ou la masse de la substance, en particulier de l'hémoglobine (Hb) dans le sang, ou des variations de ceux-ci, ou dans lequel le moyen prévu pour obtenir une valeur consiste en un tel moyen prévu pour mesurer ou calculer.

7. Un dispositif pour traiter le sang d'un patient comprenant au moins un appareil selon l'une quelconque des revendications précédentes.

8. Un dispositif selon la revendication 7, adapté au traitement d'un patient par dialyse, hémofiltration, ultrafiltration, et/ou hémodialyse.

Patient: 94; BV (OH=0): 4.3 L; m_Hb: 521g; K_Guyton: 1:5.0;

$$Hb = \frac{1}{b + a \cdot OH}$$

# Fig. 1

**Fig. 2**

Patient: 94; BV (OH=0): 4.3 L; m_Hb: 521g; K_Guyton: 1:5.0;

**Fig. 3**

**Fig. 4**

Fig. 5

Fig. 6

$$ECW = ECW_{blood} + ECW_{interstit} = 20.0\ L$$

$$ICW = ICW_{blood} + ICW_{interstit} = 30.0\ L$$

$$TBW = ECW + ICW = 50.0\ L$$

Fig. 7

Fig. 8

**Fig. 9**

**EP 2 380 609 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0247609 A2
- WO 2004022135 A1
- WO 2007140993 A1
- WO 9823311 A1
- US 20070215545 A1
- EP 1927371 A2
- WO 2006002685 A1
- WO 9219153 A

**Non-patent literature cited in the description**

- **NADLER, S.B. ; HIDALGO, J.U. ; BLOCK, T.** Prediction of Blood Volume in Normal Human Adults. *Surgery,* 1962, vol. 51, 224-232
- **DRUKKER ; PARSON ; MAHER.** Replacement of Renal Function by Dialysis. Kluwer Academic Publisher, 2004, 397-401